# EUROPEAN PATENT SPECIFICATION

(11) **EP 3 774 312 B1**
(45) Date of publication and mention of the grant of the patent: **11.12.2024**
(21) Application number: 18913110.5
(22) Date of filing: 30.03.2018
(51) Int. Cl.: B29D 22/00, B32B 1/08, B32B 27/18

(54) **PACKAGE FOR PVC PRODUCTS**
VERPACKUNG FÜR PVC-PRODUKTE
EMBALLAGE POUR PRODUITS EN PVC

(43) Date of publication of application: 17.02.2021
(73) Proprietor: Amcor Flexibles North America, Inc., Neenah, WI 54956 (US)
(72) Inventor: LIU, Yuan, Neenah, WI 54957 (US); MICHAUD, Ryan A., Neenah, WI 54957 (US)
(74) Representative: Vossius & Partner Patentanwälte Rechtsanwälte mbB
(86) International application number: PCT/US2018/025478
(87) International publication number: WO 2019/190560

(56) References cited:
- WO-A1-2016/165942
- US-A- 5 454 806
- US-A1- 2002 115 975
- US-A1- 2008 311 370
- US-A1- 2009 208 685
- US-A1- 2012 233 965
- US-A1- 2017 158 400
- TOPAS ADVANCED POLYMERS: "TOPAS Packaging", PRODUCT BROCHURE MEDICAL BROCHURE DIAGNOSTIC BROCHURE OPTICAL BROCHURE, 1 April 2011 (2011-04-01), XP055606572
- WWW.TOPAS.COM: "TOPAS PACKAGING", 1 April 2011 (2011-04-01), pages 1 - 32, XP055523781, Retrieved from the Internet <URL:https://topas.com/sites/default/files/files/Packaging_E_2014-06.pdf> [retrieved on 20181114]
- TOPAS: "Cyclic Olefin Copolymer (COC)", TOPAS ADVANCES POLYMERS - BROCHURE, 1 January 2011 (2011-01-01), pages 1 - 32, XP055580835, Retrieved from the Internet <URL:www.topas.coni> [retrieved on 20190415]
- BURGOS N ET AL: "Degradation of poly(vinyl chloride) plasticized with non-phthalate plasticizers under sterilization conditions", POLYMER DEGRADATION AND STABILITY, BARKING, GB, vol. 94, no. 9, 1 September 2009 (2009-09-01), pages 1473 - 1478, XP026348697, ISSN: 0141-3910, [retrieved on 20090518]
- KAREN BERGER ET AL: "Achieving Efficacy and Sterility in Flexible Packaging", MEDICAL DEVICE AND DIAGNOSTIC INDUSTRY, CANON COMMUNICATIONS, SANTA MONICA, CA, US, 2 January 2004 (2004-01-02), pages 1 - 9, XP002478228, ISSN: 0194-844X, [retrieved on 20040102]
- BERNARD L ET AL: "Migration of plasticizers from PVC medical devices: Development of an infusion model", INTERNATIONAL JOURNAL OF PHARMACEUTICS, ELSEVIER, NL, vol. 494, no. 1, 13 August 2015 (2015-08-13), pages 136 - 145, XP029276506, ISSN: 0378-5173, DOI: 10.1016/J.IJPHARM.2015.08.033

## Description

### TECHNICAL FIELD

This disclosure relates generally to protective packaging of medical products or similar products that have flexible PVC components.

### BACKGROUND

Plasticized polyvinyl chloride (PVC) is a widely utilized polymer composition. It is found in a wide range of consumer products and construction materials. Specifically many medical devices use plasticized PVC for critical components, such as tubing, balloons, or bags.

Pure PVC is a brittle material. Traditionally, plasticizers are added to the PVC to make it flexible and easily processed. Most plasticizers do not covalently bind to the PVC, resulting in the plasticizer migrating from the PVC matrix into the surrounding environment. The amount and speed of leakage of plasticizer from PVC components into surrounding mediums is influenced by a number of different factors including, but not limited to, storage time, temperature, pH, concentration, agitation, and solubility. The movement of plasticizer may alter the long term properties of the PVC material or PVC containing product as well as the media into which the plasticizer has moved. Additionally, plasticizer movement could cause interaction between the PVC product and the adjacent media causing them to stick to each other. It may then become difficult to separate the PVC containing product from the adjacent media.

Medical devices and similar products often utilized highly plasticized PVC to create products that function properly. PVC has been used to create flexible items such as tubing, catheter balloons, and blood bags. These products may be packaged in disposable packaging before use, for protection and distribution purposes. Historically, polyacrylonitrile copolymer products have been used to package PVC containing products with great success. Polyacrylonitrile copolymer provides good protection of the product and resists movement of plasticizers, preventing the PVC containing devices from changing physical properties or sticking to the package surface. Polyacrylonitrile copolymer also provides significant oxygen and moisture barrier for applications that require protection, and often do not require the addition of other barrier layers. However, reduced availability and increased costs of polyacrylonitrile copolymer have necessitated sourcing of other packaging materials. Many of the common and cost effective packaging materials do not perform as well as polyacrylonitrile copolymer and device degradation and sticking occur. In some cases, it becomes difficult to remove the product from the package at the time of use, especially in a sterile setting.
The degradation of poly(vinyl chloride) plasticized with non-phthalate plasticizers under sterilization conditions has been studied by N. Burgos and A. Jimenez in POLYMER DEGRADATION AND STABILITY 2009, vol. 94(9), pages 1473-1478. K. Berger and D. Dodrill studied the efficacy and sterility in flexible packaging in MEDICAL DEVICE AND DIAGNOSTIC INDUSTRY, 20040102), pages 1-9. WO 2016/165942 A1 discloses a tube for a medical container. The migration of plasticizers from PVC medical devices has been studied by L. Bernard et al. in INTERNATIONAL JOURNAL OF PHARMACEUTICS, 2015, vol. 494(1), pages 136-145.

### SUMMARY

Plasticized polyvinyl chloride containing medical devices tend to stick to packaging materials after extended storage, making the devices difficult to remove from the package without increased contamination or damage risks. Additionally, the plasticizers in the polyvinyl chloride can migrate to the packaging material, changing the physical properties of the polyvinyl chloride component and the packaging materials, potentially shortening the effective shelf life of the packaged product. Disclosed herein are descriptions of embodiments of packaged products that overcome these issues.

Some embodiments of the packaged product use a thermoformed tray containing a cyclic olefin copolymer that has a glass transition temperature greater than 40°C. A product comprising flexible polyvinyl chloride is in the tray and at least the flexible polyvinyl chloride portion of the product in direct contact with the cyclic olefin copolymer of the tray. Advantageously, after the packaged product is stored for at least 5 days at 55°C and 90% RH, the product releases from the cyclic olefin copolymer when the tray is inverted.

In some embodiments, the packaged product also comprises a lid connected to the thermoformed tray. The lid and thermoformed tray may be connected by a heat seal. The packaged product may be hermetically sealed.

In some embodiments the packaging components, such as the lid or thermoformed tray, comprise a breathable material. The packaging components may comprise a barrier polymer.

The product in the packaging may be a medical product. The product may have been sterilized.

In some embodiments, the packaged product comprises a lid, a thermoformed tray and a product. The product is located between the lid and the thermoformed tray and is comprised of flexible polyvinyl chloride polymer. The thermoformed tray is comprised of a heat sealable product contact layer comprising cyclic olefin copolymer, at least one barrier layer, at least one inner layer comprising polyamide, and a layer comprising polyester that is greater than 75% of the total thickness of the thermoformed tray. The at least one inner layer of polyamide is adjacent to the barrier layer. The lid and the thermofonned tray may be connected by a peelable heat seal proximate the perimeter of the thermoformed tray. There may also be a barrier layer in the lid. The polyvinyl chloride polymer of the product is in direct contact with the heat sealable product contact layer and the polyvinyl chloride polymer releases from the heat sealable product contact layer of the tray under gravitational forces.

In some embodiments of the packaged product, the barrier layer of the thermoformed tray comprises ethylene vinyl alcohol copolymer. In some embodiments, the cyclic olefin copolymer of the heat sealable product contact layer of the thermoformed tray has a glass transition temperature of at least 40°C.

In some embodiments, the packaged product has at least one packaging component comprising a heat sealable product contact layer comprising a cyclic olefin copolymer, the cyclic olefin copolymer having a glass transition temperature of at least 40°C. The product comprises flexible polyvinyl chloride polymer and the polyvinyl chloride polymer is in direct contact with the heat sealable product contact layer of the packaging component. The product releases from the heat sealable product contact layer after the packaged product is aged for at least 5 days at 55°C and 90% RH.

In some embodiments, the packaged product is hermetically sealed. In some embodiments, the glass transition temperature of the cyclic olefin copolymer is between about 40°C and about 140°C.

Some embodiments of the packaged product further comprise an oxygen absorber. In some embodiments of the packaged product, the packaging component further comprises an oxygen absorber catalyst.

### BRIEF DESCRIPTION OF THE DRAWINGS

This disclosure may be more completely understood in consideration of the following detailed description of various embodiments of the disclosure in connection with the accompanying drawings, in which:
**Figure 1** is an exemplary embodiment of a packaged product;
**Figure 2** is a partial cross-sectional view of an exemplary embodiment of a packaged product; and
**Figure 3** is an exemplary embodiment of a packaged product.

The drawings show some, but not all, embodiments. The elements depicted in the drawings are illustrative and not necessarily to scale, and the same reference numbers denote the same (or similar) features throughout the drawings.

### DETAILED DESCRIPTION

Disclosed herein is a package that can be useful for packaging medical devices, or similar products, that are made of or have components made of flexible polyvinyl chloride (PVC). The packaging disclosed resists sticking to the medical device components and the product can be easily removed from the package without excessive effort (such as violent shaking or use of a secondary tool) or risk of damage. The product contact layer of the packaging component comprises a material that can be hermetically sealed, exhibits low uptake of typical PVC plasticizer materials and does not stick to the PVC material after an extended storage period. The product contact layer is a cyclic olefin copolymer with a glass transition temperature of at least 40°C and is preferably part of a thermoformed tray packaging component.

In the past, polyacrylonitrile copolymer materials have been used for packaging medical device products that require protection. These materials performed well with respect to protecting and maintaining the integrity of flexible PVC materials. The plasticizing components of the PVC did not have a tendency to enter into the polyacrylonitrile copolymer packaging components. Thus, both the PVC component flexibility and the packaging material barrier had long term stability. The PVC containing products did not stick to the polyacrylonitrile copolymer packaging. However, polyacrylonitrile copolymer has become difficult to source and other packaging solutions are required.

Standard packaging materials have been tested as a replacement to the polyacrylonitrile copolymer. Polyethylene based materials, such as linear low density polyethylene or ethylene vinyl acetate copolymers perform well as heat sealable material and can be combined with barrier materials to produce protective packaging components. However, it has been shown that the plasticizer materials found in the PVC products easily move into and through these polyethylene based materials. This can have several deleterious effects. First, the PVC component can lose flexibility over time due to the loss of plasticizer. This results in a shortened shelf life for the PVC containing products to deliver adequate performance. Second, the plasticizers potentially move through the polyethylene and into to various portions of the packaging material, such as the barrier materials, degrading the barrier performance of the overall packaging structure. Third, the movement of the plasticizer components can cause enough chemical interaction between the PVC containing component and the packaging material that the two materials stick to each other. This can make it difficult to remove the product from the package.

Surprisingly and advantageously, the present inventors have found that some polyethylene copolymers based on ethylene and norbomene exhibit excellent resistance to the movement of the plasticizer from the PVC components. Packages produced with a product contact layer of ethylene norbornene copolymers of the type disclosed herein can minimize or eliminate any tackiness between the PVC and the package.

Before describing several exemplary embodiments, it is to be understood that the packaged product is not limited to the details of construction or alternatives set forth in the following description. The packaged product is capable of other embodiments and of being practiced or being carried out in various ways.

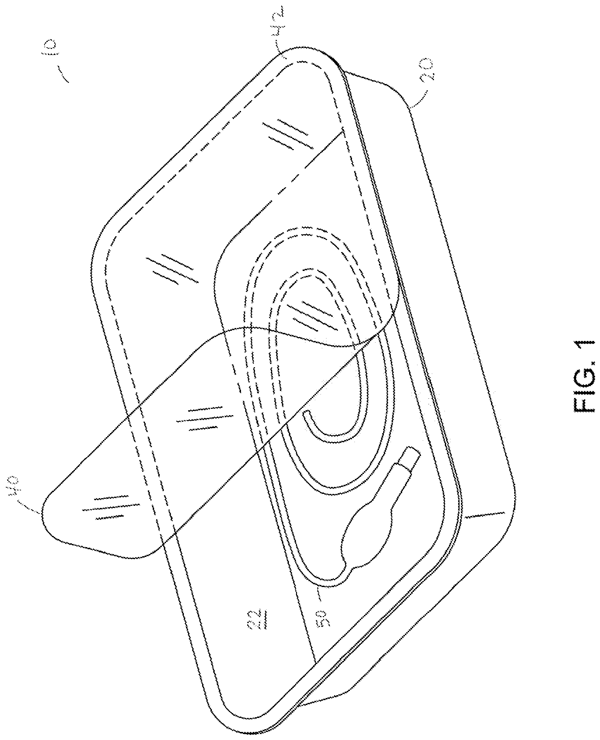

Referring to the drawings. Figure 1 represents an example of a packaged product **10.** A product **50** is located inside of a thermoformed tray **20** such that the product **50** is in contact with a product contact layer **22** of the thermo formed tray **20.** A lid **40** is connected to the tray by a seal **42** at or near the periphery of the thermoformed tray **20.** Figure 1 shows how the seal **42** of the lid **40** may be peeled open such that the product **50** can be removed.

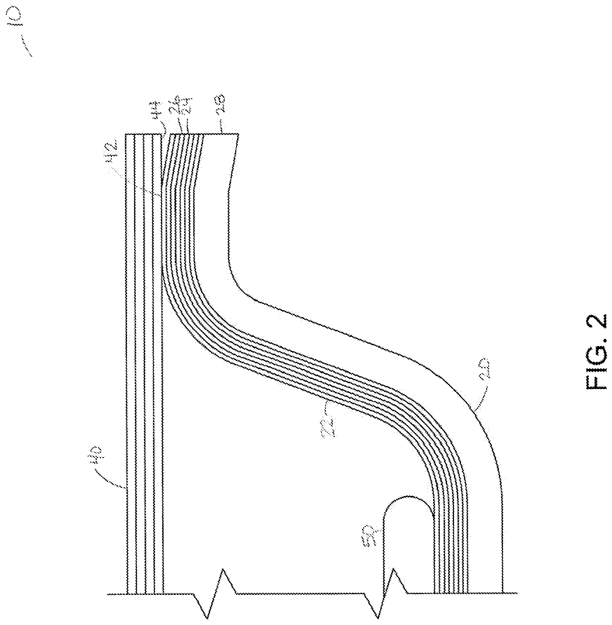

Figure 2 is a cross-sectional view of an embodiment of a packaged product **10.** A product **50** is located inside of a thermoformed tray **20** such that the product **50** is in contact with a product contact layer **22** of the thermoformed tray **20.** A multilayer lid **40** is connected to the tray **20** by a seal **42** near the perimeter of the thermoformed tray **20.** A portion of the lid **40** exterior to the seal **42** may be unsealed **44.** This allows a user to manually grab the unsealed portion **44** of the lid **40,** peel the lid **40** back by exerting force, and gain access to the product **50.** In this embodiment, the thermoformed tray **20** has a barrier layer **24,** at least one inner layer adjacent to the barrier layer **26,** and an exterior layer **28.**

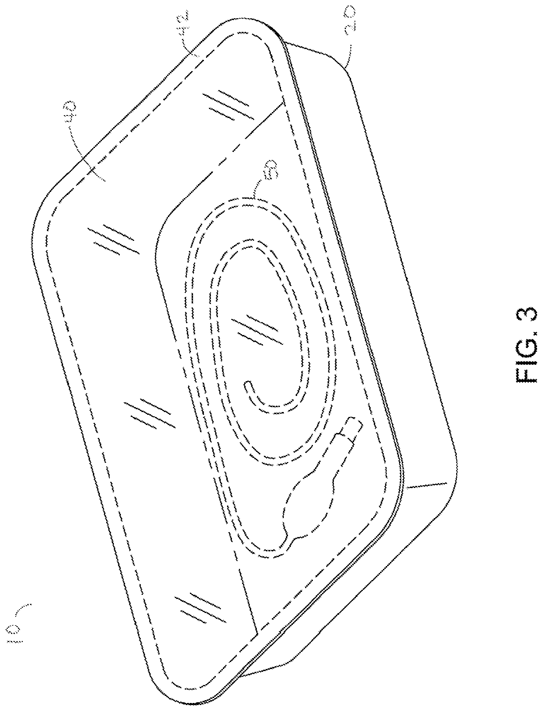

Figure 3 represents an example of a packaged product **10.** A product **50** is located inside of a thermoformed tray **20** such that the product **50** is in contact with a product contact layer of the thermoformed tray **20.** A lid **40** is connected to the tray by a seal **42** at the periphery of the thermoformed tray **20.** Figure 3 shows that the product **50** may be hermetically sealed inside the package, between the thermoformed tray **20** and the lid **40.**

### Definitions

As used herein, the term "heat sealable" refers to components or materials that are amenable to being bonded to another similar or dissimilar component or material by a heat seal. The term "heat seal" refers to bonding two or more materials by the application of heat and pressure over a relatively short dwell time. A heat seal is generally formed on a package component to attach components to themselves or other components. The heat seal may be sufficient in size, shape and strength to create a hermetic package that is durable through distribution and storage of the package. As used herein, the term "peelable seal" or "peelable heat seal" refers to connections between packaging components that are of a strength that can be manually peeled open (i.e. opened by peeling the components apart manually and without assistance from an auxiliary instrument). A peelable seal may have an initial opening force of less than about 2,500 g/inch when sealed and tested in accordance with ASTM F88. Preferably, a peelable seal may have an initial opening force between about 0 g/inch and 2,000 g/inch when tested in accordance with ASTM F88.

As used throughout this application, the term "package" refers to any component or combination of components used to wholly or partially surround a product. A package may take many, various forms. For example, the term "package" may include pouches that wholly surround a product (or products) to be packaged; the term "package" may also include films, and sheets that partially surround a product (or products) to be packaged and, when used in conjunction with another film, or sheet wholly surround a product (or products). In various embodiments of the present application, the film, sheet, etc. may be thermoformed into a cup, tray, plate or other form.

As used throughout this application, the term "thermoformed" refers to polymer film or sheet formed into a desired shape by the application of a differential pressure between the film or sheet and a mold, by the application of heat, by the combination of heat and the application of a differential pressure between the film or sheet and a mold, or by any thermoforming technique known to those skilled in the art.

As used throughout this application, the term "product contact layer" or "interior layer" refers to a layer of a packaging component such as a film, sheet, or thermoformed tray involved in packaging a product. This layer is oriented toward the product and is located such that when a product is placed in the package, it becomes possible for the product to contact this layer. If the packaging component is multilayer, the product contact layer is the innermost layer of a closed package, relative to a product in the filled package (i.e. the inside of the package is generally lined with a product contact layer). If the packaging component is monolayer, the product contact layer may also be the exterior layer.

As used herein, the term "inner layer" refers to one or more layers of a multilayer material that is adjacent to another layer on both sides. The surfaces of an inner layer are covered by another layer of the multilayer material.

As used throughout this application, the term "exterior layer" refers to a layer comprising the outermost surface of a film, sheet, thermoformed tray or other packaging component relative to a product in the filled package (i.e., the layer of the film, sheet or other article farthest from a product of the filled package).

As used herein, the term "barrier layer" is a layer of a packaging component comprised of materials that provide protection to the packaged products. Barrier layers may prevent or reduce the ingress or egress of gasses, moisture, light or other things. Barrier layers may be coextruded, printed, coated or incorporated into the packaging component by any process known to those skilled in the art.

As used herein, the term "polyamide" means a high molecular weight polymer having amide linkages (-CONH-)ₙ which occur along the molecular chain, and includes "nylon" resins which are well known polymers having a multitude of uses including utility as packaging films, bags, and casings.

As used herein, the term "polyester" refers to synthetic homopolymers and copolymers having ester linkages between monomer units which may be formed by condensation polymerization methods. Polymers of this type are preferable aromatic polyesters and more preferable, homopolymers and copolymers of polyethylene terephthalate), poly(ethylene isophthalate), poly(butylene terephthalate), polyethylene naphthalate) and blends thereof. Suitable aromatic polyesters may have an intrinsic viscosity between 0.60 to 1.0, preferably between 0.60 to 0.80.

The term "adhesive layer," or "tie layer," refers to a layer or material placed on one or more layers to promote the adhesion of that layer to another surface. Preferably, adhesive layers are positioned between two layers of a multilayer film to maintain the two layers in position relative to each other and prevent undesirable delamination. Unless otherwise indicated, an adhesive layer can have any suitable composition that provides a desired level of adhesion with the one or more surfaces in contact with the adhesive layer material.

As used herein, the term "flexible polyvinyl chloride" or "flexible polyvinyl chloride polymer" refers to polyvinyl chloride (PVC) polymer that has had plasticizers added to modify the physical properties. Plasticizers are additives blended into another material with the purpose of decreasing the viscosity of the material. Plasticizers can be solids or liquids that interrupt the network of polymer chains, decreasing the chain-to-chain attraction. This leads to a more flexible polymer material. Pure PVC materials are rigid, but become extremely flexible and durable with the addition of plasticizers. PVC becomes useful in many applications once plasticized.

In general, plasticizers used in PVC products are phthalates, most commonly phthalate esters. Plasticizers commonly used in PVC for medical products are bis(2-ethylhexyl) phthalate (DEHP) and bis(2-ethylhexyl) benzene-1,4-dicarboxylate (DEHT).

As described herein, the packaged products have packaging components with properties that allow for the product to release or separate easily from the product contact layer of the packaging component, even after aging at elevated temperatures. Optimally, the product separates from the product contact layer of the packaging component when the package is inverted in such a way that gravitational forces cause the product to fall away from the product contact layer. The product will fall away from the product contact layer absent any other constraints, such as a lid or locking mechanism. In other words, the weight of the product is sufficient to cause the product to fall away from the product contact surface absent any physical constraints. In some cases, the product may exhibit a small amount of tackiness to the surface, but still fall away under inversion. Tackiness to the extent that the product only releases from the product contact surface under agitation of the package, is not recognized as acceptable release. In the worst cases of poor release properties, the removal of the products may require significant force by pulling or some other manner, and could result in a damaged product.

The phrase "the product releases from the cyclic olefin copolymer when the tray is inverted" as used herein means that the absent any physical means of constraint, the weight of the product is sufficient to move the product away from the cyclic olefin copolymer of the product contact surface after the packaging component has been inverted. No agitation of the packaging component or other assistance is required to move the product away from the packaging component. Similarly, the phrase "the product releases from the heat sealable product contact layer" as used herein means that there is no tackiness between the product and the heat sealable product contact layer. The user can remove the product from the package without any detection of sticking.

The phrase "glass transition temperature" as used herein means the temperature at which a polymer changes from an amorphous glassy state to a rubbery state, and in the transition region, various properties such as an elastic modulus, an expansion coefficient, a heat content, a refractive index, a dielectric constant, and so forth, are changed. The glass transition temperature can be measured from the change of these properties, but more definitely, this can be evaluated by a known method by using differential scanning calorimetry (DSC) or dynamic mechanical analysis (DMA). When measuring the glass transition temperature of a polymer by DSC, the glass transition temperature can be determined using ISO 11357-1, -2 and -3 test methods.

As used herein, "hermetic package" or "hermetically sealed" refers to a package that is closed in some manner such that the inside of the package and the product therein are isolated from the environment outside of the package. Generally, sealed hermetic packages are significantly airtight and moisture proof. For medical products, hermetic packages are often maintaining a sterile environment inside. preventing ingress of bacteria or other living microorganisms.

### Alternative Embodiments

The packaged products disclosed herein could include packaging components in a wide variety of formats. Some embodiments of the packaged products use a tray and lid format, as is common for medical product packaging. The package may take the form of a pouch using relatively flexible materials. The packaged products may include one, two, three or more packaging components including rigid and flexible materials. The packaging components may be polymeric and may be composites of polymers and other materials, such as paper and metal. The packaged products may include functional components such as labels or headers. Additional components may be added to the packaged products such as inserts or oxygen absorbing packets. The embodiments described herein are not limiting to the claims.

The package provides protection to the product within. Preferably, the package provides a hermetic barrier between the product and the environment outside of the package, until the point at which the packaged is opened. In some embodiments the product has been sterilized after it has been hermetically sealed inside the package and the package prevents bacteria and other organisms from contaminating the product. The product may be sterilized after packaging by any means known, including gamma ray irradiation or ethylene oxide gas.

If gas sterilization is used as a method to sterilize the packaged product, the package may require a breathable portion. The breathable portion allows the entry of the sterilizing gas but prevents transmission of bacteria.

It may be desirable for the package to have high barrier to oxygen and/or moisture. This protects the product from degradation over the shelf life. The packaging components should be manufactured from a material that provides sufficient barrier. The necessary barrier may be provided by a single layer of the packaging material. The packages disclosed herein provide examples of varying barrier properties and it should be understood that the barrier of the packaging should be modified to meet the requirements of any specific application. In a preferred embodiment, the package provides oxygen and moisture barrier.

In some embodiments, the package is hermetically sealed around the product In some embodiments the package is hermetic through storage and distribution, and the hermeticity is broken just prior to the time that the product is to be used. Hermeticity of the package can be broken in any number of ways, including but not limited to cutting, tearing the packaging components. In some embodiments the seals are peelable and the user can manually peel the packaging components apart, breaking the hermeticity of the package and gaining access to the product inside.

In addition to providing protection to the product via hermeticity and barrier properties, the packaging material may perform other functions. The packaging materials may be printed or otherwise labeled in conventional ways to deliver marketing messages, product descriptions, opening instructions, pictorials, or other various information. The packaging materials may provide active functionality such as, but not limited to, oxygen absorption or self-sterilization. The active functionality may be incorporated into the main packaging components, or may be introduced through inserts, patches or other secondary components. The active functionality may be triggered by any means, including influences inside the package (i.e., headspace conditions such as gas composition or humidity) or outside the package (e.g., ultraviolet radiation).

The packaging materials disclosed herein are typically one-time-use and disposable. The packaging materials may be suitable for recycling after use. Alternatively, the packaging may be in a format that is suitable for multi-use or repeated use.

In some embodiments the product can be removed from the package by peeling the packaging components apart at the seal. In this case, the packaging materials are formulated and/or the sealing conditions are selected such that the seal is strong enough to remain closed during handling and distribution of the package, but weak enough that it can be breached by manual force. These packaging formulations and sealing conditions can be any that are known in the art to produce a peelable seal. In some embodiments, the packaged product includes an area of the packaging components, exterior to the seal, which is unsealed. This unsealed portion, located further toward the perimeter of the packaging components than the heat seals, may take the form of a peel tab, allowing a user to grab the packaging components, gaining leverage for peeling open.

Alternatively, the packaging components can be connected in such a way that the seals are not peelable and cannot be manually separated. In this case, the package may have tear notches, scoring or other features to provide opening ease. In some embodiments, the seals may not be peelable and the package must be tom, cut or otherwise breached to gain access to the product inside.

In some embodiments, the packaged product includes a thermoformed tray. The thermoformed tray may consist of generally polymeric materials. The thermoformed tray may be a composite of materials including, but not limited to, polymers, cellulosic materials, non-woven materials, or metals. The materials generally need to be of the type that are conducive to the thermoforming process. Upon heating and application of pressure, the material must form and then retain the new shape provided during the thermoforming process. In some embodiments the thermoformed tray is relatively rigid, and in other embodiments the thermoformed tray is relatively flexible. The thermoformed tray may be formed by any process known for producing formed packaging components.

Some embodiments of the packaged product incorporate a thermoformed tray that has a flat sealing area, or flange, generally surrounding the perimeter. The flange creates a flat area against which a lid may be connected. In some embodiments a lid is heat sealed to the thermoformed tray at the flange area. Alternatively, other packaging components, such as a second thermoformed tray, may be connected to the thermoformed tray.

The packaging components described herein have a product contact layer containing ethylene norbomene copolymer, which is a cyclic olefin copolymer (COC). COC materials are commercially available from Topas as amorphous, transparent copolymers of ethylene with norbomene made by polymerization with a metallocene catalyst. These commercially available COC materials reportedly have high transparency and gloss, excellent moisture barrier and aroma barrier properties, high stiffness, high strength, excellent biocompatibility and inertness and are easy to extrude and thermoform. The COC materials are available with varying glass transition temperatures. COC materials have previously been used for pharmaceutical, medical and food packaging applications, including use in coextruded cast films for blister packaging, and may be blended with polyethylene. The product contact layers of the packaging components described herein include ethylene norbomene copolymers having a glass transition temperature (Tg) of 40-140°C. In some embodiments, the product contact layers of the packaging components described herein comprise polymeric units derived from essentially only ethylene and norbomene comonomers.

In various embodiments, the product contact layer may also function as a heat sealing or heat sealable layer to facilitate formation of hermetically sealed packages. The product contact layer comprises at least 90 wt.% of ethylene norbornene COC, more preferably at least 95 wt. %, and most preferably 100 wt. %. It may be blended with up to 10 wt. %. preferably up to 5 wt. % and more preferably up to 2.5 wt.% of compatible polymers such as polyolefins (e.g., polyethylene, LLDPE, EAO copolymers, LDPE), colorants, processing aids and the like. Use of these polymers and components in a blend with the COC may have a negative effect on the properties of this layer and addition of amounts above 10 wt.% may be unacceptable for many applications.

The product contact layer is on the interior surface of the packaging component. The product contact layer may be the only layer of the packaging component, such that it is also the exterior layer of the packaging component. Alternatively, the product contact layer may be coupled with other layers that provide the necessary properties, such as barrier. In some embodiments, the product contact layer is part of a multilayer component and has a thickness of about 1 mil. The thickness of the product contact layer may be less than 1 mil, less than 0.5 mil or less than 0.25 mil.

In some embodiments, one or more of the packaging components (lids, thermoformed trays, etc.) may include a barrier layer. Typically, barrier layers prevent the transmission of light, moisture, oxygen or other gases through the packaging. Barrier layers may also protect other layers from migration from package contents (for example, oils). Barrier layers may be incorporated by any known means including, but not limited to, coextrusion, lamination, coating, printing and vacuum coating. If included, a barrier layer preferably functions both as a gas barrier layer, and as a moisture barrier layer, although these functions may be provided by separate layers. A gas barrier layer is preferably an oxygen barrier layer, and is preferably an inner layer positioned between and protected by surface layers (i.e., product contact and exterior layers). The barrier layer may comprise a thermoplastic oxygen barrier of ethylene-vinyl alcohol copolymer (EVOH).

If included, an oxygen barrier is preferably selected to provide an oxygen permeability sufficiently diminished to protect the packaged article from undesirable deterioration or oxidative processes. For example, a packaging component may comprise an oxygen barrier having an oxygen permeability that is low enough to prevent oxidation of oxygen sensitive products and substances to be packaged. In some embodiments, a packaging component will have an oxygen transmission rate (O2TR) of less than 1, preferably less than 0.1, more preferably less than 0.01, and most preferably less than 0.001 g/100 inches²/24 hours when tested at 23°C and 50% relative humidity using ASTM F1927-14 Standard Test Method for Determination of Oxygen Gas Transmission Rate, Permeability and Permeance at Controlled Relative Humidity Through Barrier Materials Using a Coulometric Detector (2014)

If included, a moisture barrier is preferably selected to provide a moisture permeability sufficiently diminished to protect the packaged article from undesirable deterioration. For example, a film may comprise a water barrier layer having a moisture permeability that is low enough to prevent deleterious effects upon packaged products. In some embodiments, a film has a water vapor transmission rate (WVTR) of less than 0.01g/100 inches² per 24 hours at Room Temperature (RT) (23°C) and 1 atmosphere, or less than 0.001g/100 inches2 per 24 hours at Room Temperature (RT) (23°C) and 1 atmosphere when tested using ASTM F1249 Standard Test Method.

A barrier layer can comprise any suitable material. An oxygen barrier layer can comprise EVOH, polyvinylidene chloride, polyamide, polyester, polyalkylene carbonate, polyacrylonitrile copolymer, nanocomposite, a metallized film such as aluminum vapor deposited on a polyolefin, as known to those of skill in the art. One suitable EVOH barrier material is a 44 mol % EVOH resin E151B sold by Eval Company of America, under the trade name Evai^{®}LC-E151B. Another example of an EVOH that may be acceptable can be purchased from Nippon Gohsei under the trade name Soarnol^{®} AT (44 mol% ethylene EVOH). Suitable moisture barrier layers include aluminum foil, PVDC, or polyolefins such as LDPE or LLDPE. It is desirable that the thickness of the barrier layer be selected to provide the desired combination of the performance properties sought e.g. with respect to oxygen permeability, and delamination resistance, and water barrier properties. Greater thicknesses may be employed. However oxygen barrier polymers tend to be relatively expensive and therefore it is expected that less costly resins will be used in other layers to impart desirable properties once a suitable thickness is used to achieve the desired barrier property for the film layer combination. For example, the thickness of an oxygen barrier layer may advantageously be less than about 0.45 mil (10.16 microns) and greater than about 0.05 mil (1.27 microns), including approximately 0.10, 0.20, 0.25, 0.30, 0.40, or 0.45 mil thick.

The packaging components may include any number of inner layers. These inner layers may have the function of enhancing adhesion, barrier, durability, or appearance. Between any of the layers, a tie or adhesive layer may be provided to provide adhesion and continuity between the layers.

Some embodiments include inner layers that comprise polyamide. The polyamide may comprise nylon 66, nylon 610, nylon 66/610, nylon 6/66, nylon 11, nylon 6, nylon 66T, nylon 612, nylon 12, nylon 6/12, nylon 6/69, nylon 46, nylon 6-3-T, nylon MXD-6, nylon MXDI, nylon 12T and nylon 6I/6T or blends thereof. More than one layer of polyamide may be used. Preferably, there are two inner layers of polyamide, located on either side of an EVOH barrier layer.

The packaging described herein includes an exterior layer. Since it is visible to the user/consumer, in both monolayer and multilayer embodiments, the exterior surface of the film preferably has desirable optical properties and may preferably have high gloss. Also, it preferably withstands contact with sharp objects and provides abrasion resistance, and for these reasons it is often termed the abuse resistant layer. The exterior layer is subject to handling and abuse e.g. from equipment during packaging, and from rubbing against other packages and shipping containers and storage shelves during transport and storage. This contact causes abrasive forces, stresses and pressures which may abrade away the film causing defects to printing, diminished optical characteristics or even punctures or breaches in the integrity of the package. Therefore the exterior surface layer is typically made from materials chosen to be resistant to abrasive and puncture forces and other stresses and abuse which the packaging may encounter during use. The exterior layer should be of suitable stiffness, flexibility, flex crack resistance, modulus, tensile strength, coefficient of friction, printability, and optical properties. The exterior layer may be tough to impart resistance to opening by children e.g. preventing the package from being opened by a child's bite. A preferred exterior layer comprises polyester, which has excellent durability, thermoformability and appearance. Polyester also has economic advantages over many other polymers. The exterior layer thickness is typically 2 to 50 mils. Thinner layers may be less effective for abuse resistance, however thicker layers, though more expensive, may advantageously be used to produce films having unique highly desirable puncture resistance and/or abuse resistance properties.

In some embodiments, the exterior layer thickness is less than 50% of the overall packaging thickness. In some embodiments, the packaging component is a thermoformed tray and the thickness of the exterior layer is greater than 75% of the overall tray thickness.

Some embodiments of the packaged product may include a lid. The lid may be connected to the thermoformed tray or any other packaging component, including itself. The lid may be relatively thin and flexible or may be rather rigid and even thermoformed. The lid may be clear or opaque, and may be printed or labeled. The lid may contain oriented films such as oriented polyethylene terephthalate (OPET) or metalized films such as metalized oriented polyethylene terephthalate. The lid may contain a barrier layer. The lid may be connected to the thermoformed tray in a seal that follows the perimeter of the tray and the lid may be sized such that there is excess material beyond the seal. This excess material remains unsealed and provides a peel tab portion.

The packaging components of certain embodiments are connected by a seal. The seal may be created by any known process including, but not limited to, heat sealing, rf welding, or ultrasonic sealing. The seal is typically located around the perimeter of the packaging materials and provides a hermetic package upon closing. The seal may be a heat seal that is proximate the perimeter of the packaging components. The seal may be made in a position such that there is a portion of the packaging materials outside of the seal area that is unsealed, providing a peel tab. A peel tab should be included if the seals are manually peelable.

The product packaged may be a medical device, a medical supply, an industrial product, a personal care item, a one-time use product, a reusable product, a disposable product or a durable product. The product has at least a portion that comprises flexible (i.e., plasticized) PVC polymer. Under packaged conditions, the product is in contact with the product contact layer of at least one packaging component. In some embodiments, the flexible PVC polymer is in contact with the product contact layer. In some embodiments, the flexible PVC polymer of the product is in direct contact with the product contact layer of the thermoformed tray. In a preferable embodiment, the flexible PVC polymer of the product is in direct contact with the COC polymer of the product contact layer of the packaging, and after a duration of storage, the product does not exhibit tackiness to the packaging.

The product comprises a flexible PVC portion. The product may be made entirely of flexible PVC or may contain flexible PVC in only a small portion. Products that contain flexible PVC include, but are not limited to, blood bags, flexible medical tubing, catheters and tracheotomy devices.

One advantage of the disclosed packaged products is that the migration of the plasticizer from the PVC portion of the product into the packaging components is limited. By limiting the movement of the plasticizer, the product retains the physical properties necessary for product use, and the packaging materials retain the protective integrity. The packaging materials do not experience a change in physical properties or barrier and are less prone to delamination quality issues when the plasticizer uptake is limited or prevented. The PVC products do not experience degradation in flexibility or discoloration due to loss of plasticizer. Overall, minimization of the movement of plasticizer from the packaged product to the packaging components allows for extended shelf life of the product and package system (i.e., the system remains useful over longer storage periods).

Another advantage of the disclosed packaged products is that the products do not stick to the packaging materials. In some embodiments the product has no affinity for the product contact layer of the packaging materials, even after extended direct contact at elevated temperatures. The product will fall away from the product contact layer of the packaging components when the packaging is inverted. In other words, when the package is oriented such that gravitational forces can act upon the weight of the product in a direction opposed to the packaging, the product falls away from the packaging. In some cases, the product falls away from the package after inversion and very slight agitation (shaking) of the packaging component. In all cases, the removal of the product from the packaging, either by gravitational force after inversion or by manual selection (i.e., picking up the product with the hand), does not require excessive manual force or use of a secondary implement. In all cases, neither the product nor the packaging are damaged due to the removal of the product.

### Examples

### Plasticizer Movement Study

A plastisol was produced by mixing 2.0 g of bis(2-ethylhexyl) phthalate plasticizer (DEHP) with 3.0 g of polyvinyl chloride (PVC) polymer (3102B-C018F from Colorite) powder in a glass blender for 5 minutes. The plastisol was transferred to a two-inch diameter foil baking cup. The baking cup with plastisol was heated in an oven at 200°C for 20 minutes. The cup and mixture were cooled at room temperature for about 30 minutes, creating flexible PVC plates which were removed from the foil cup.

Packaging polymers (polymeric media) of interest were polyacrylonitrile copolymer (Barex^{®} from INEOS), ethylene vinyl acetate copolymer (EVA, 5% VA), amorphous polyester (Eastapak^{™} Polymer 9921) and various packaging film grades of cyclic olefin copolymer (Topas Advanced Polymers, see Table 1). A sample of each of these polymers was formed into a thin film and cut into disks of 0.25 inch diameter. These polymeric media disks were placed between two of the flexible PVC plates and a 20 pound weight was placed on top of the sandwich to create intimate contact between the plasticized PVC and the polymeric media disks. The entire unit (polymer sandwich plus weight) was placed into a controlled environment of 100°F and 20% humidity for a one week period.

Once removed from the controlled environment, the polymeric media disks were separated from the plasticized PVC and tested for DEHP using thermogravimetric analysis (TGA). DEHP begins to evaporate at about 200°C and will be completely removed by 300°C. The TGA curve of each polymeric media disk was subtracted from the TGA curve of a "control" disk of the same polymer (a disk that had not been exposed to the PVC plates). This calculation resulted in the amount of plasticizer that had moved from the PVC material to the polymeric media, and is shown in Table 1.

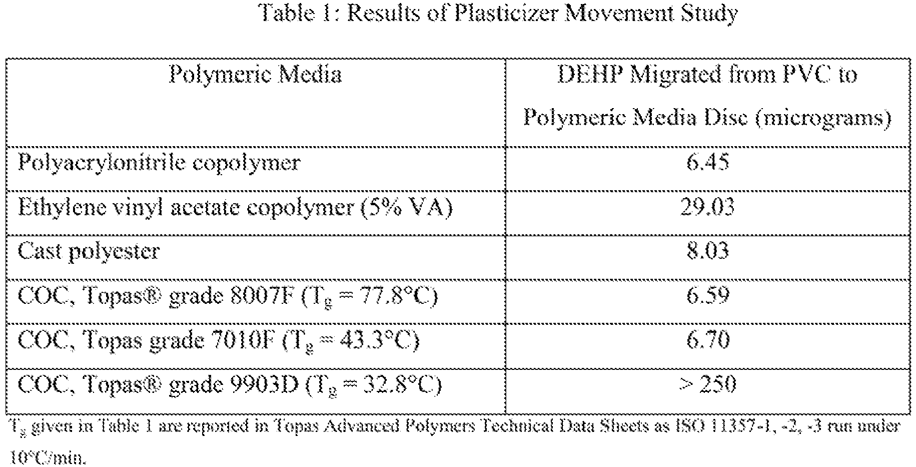

According to Table 1, cyclic olefin copolymers that are suitable for heat sealable packaging and having a glass transition temperature greater than 40°C perform similar to polyacrylonitrile copolymer in prevention of plasticizer movement.

### Plasticizer Movement and PVC Sticking

Highly plasticized PVC balloons were cut from commercial catheter systems and were placed against various packaging product contact layers. A 20 pound weight provided intimate contact between the balloon and the sealant surfaces and the samples were stored at 38°C and 0% humidity. The sealant materials studied were polyacrylonitrile copolymer (Barex^{®} from INEOS), cyclic olefin copolymer (grade 8007F, available from Topas Advanced Polymers), glycol modified amorphous polyester and LLDPE. Plasticizer transfer was measured by weight loss measured on each balloon and is reported in Table 2. Additionally, sticking was evaluated by measuring the bond between the balloon and the sealant material after three days of aging. The bonds were measured by cutting the bonded product and package to a one inch wide specimen and using a Instron^{®} type peel testing machine to measure the force required to separate the materials when pulled in a 180° orientation at a rate of about 12 in/min. The bond or peel strength values are also reported in Table 2.

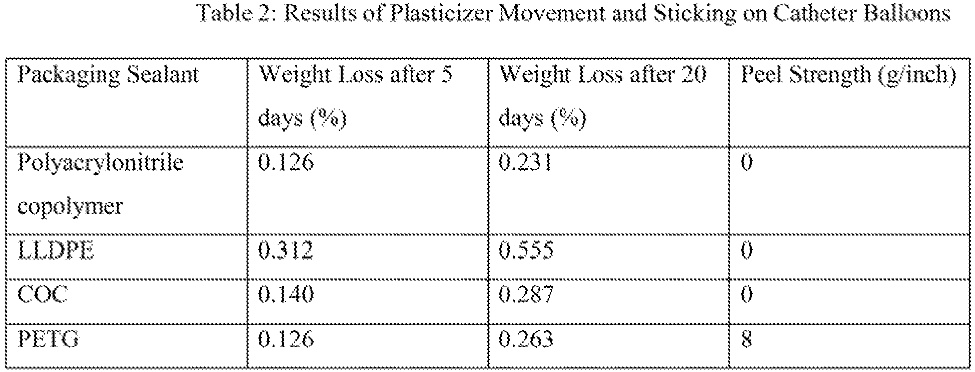

It was found that polyethylene materials such as LLDPE cause a rather large migration of plasticizer from the PVC balloon. The glycol modified polyester materials perform similar to polyacrylonitrile copolymer in plasticizer migration, but have a tendency to cause sticking. It was found that COC has acceptable performance in both plasticizer movement and sticking.

### Example 1

An exemplary package was produced and tested. A thermoformed tray was produced from a sheet of the structure 34 mil PETG / EVA blend f tie / polyamide / EVOH / polyamide / tie / COC. Referring to Figure 2, the PETG is the exterior layer **28.** the EVOH is the barrier layer **24,** the polyamide is the at least one inner layer **26** and the COC is the product contact layer **22.** The other layers of the thermoformed tray **20** are shown in Figure 2 but not labeled. The COC used for the heat sealable product contact layer was grade 8007-F, available from Topas Advanced Polymers. A catheter with a flexible PVC balloon (product **50** as shown in Figure 2) was placed into the tray such that the PVC was in direct contact with the COC heat sealable product contact layer. A lid **40** of the structure OPET / white polyethylene blend : aluminum foil / polyethylene blend was sealed to the flange **42** around the perimeter of the tray, hermetically sealing the catheter in the package. The polyethylene blend of the sealing surface was formulated to produce a peelable heat seal. The package was allowed to sit undisturbed for at least 5 days at 55°C and 90% RH. It was found that upon peeling open the lid and inverting the tray (i.e., turning the tray over), the catheter freely fell away from the thermoformed tray without shaking or agitating the package. No sticking was detected.

Although the disclosure herein has utilized reference to particular embodiments, it is to be understood that these embodiments are merely illustrative of the principles and applications. It will be apparent to those skilled in the art that various modifications and variations can be made to the method and apparatus described here without departing from the spirit and scope of the disclosure. Thus, it is intended that the present disclosure include modifications and variations that are within the scope of the appended claims and their equivalents.

## Claims

1. A packaged product comprising:
a) a thermoformed tray comprising a cyclic olefin copolymer that has a glass transition temperature greater than 40°C as determined by differential scanning calorimetry, and
b) a product comprising flexible polyvinyl chloride, wherein the flexible polyvinyl chloride comprises plasticizer, at least the flexible polyvinyl chloride portion of the product in direct contact with the cyclic olefin copolymer,
wherein after the packaged product is stored for at least 5 days at 55°C and 90% RH, the product releases from the cyclic olefin copolymer when the tray is inverted, which means that absent any physical means of constraint, the weight of the product is sufficient to move the product away from the cyclic olefin copolymer of the product contact surface after the packaging component has been inverted.

2. A packaged product according to claim 1 also comprising a lid connected to the thermoformed tray.

3. A packaged product according to claim 2 in which the lid and thermoformed tray are connected by a heat seal.

4. A packaged product according to any previous claim in which at least one of the lid or thermoformed tray comprises a breathable material, which allows the entry of a sterilizing gas but prevents transmission of bacteria.

5. A packaged product according to any other claim in which the packaged product is hermetically sealed.

6. A packaged product according to claim 1 in which the thermoformed tray further comprises a barrier polymer that provides protection to the packaged product.

7. A packaged product according to claim 1 in which the product is a medical product.

8. A packaged product according to claim 7 in which the product has been sterilized.

9. A packaged product comprising a lid, a thermoformed tray and a product,
wherein
a) the product is located between the lid and the thermoformed tray and comprises flexible polyvinyl chloride polymer, wherein the flexible polyvinyl chloride comprises plasticizer;
b) the thermoformed tray comprises:
i) a heat sealable product contact layer comprising cyclic olefin copolymer;
ii) at least one barrier layer comprised of materials that provide protection to the packaged products;
iii) at least one inner layer comprising polyamide, the at least one inner layer adjacent to the barrier layer; and
iv) a layer comprising polyester that is greater than 75% of the total thickness of the thermoformed tray; and
c) the lid and the thermoformed tray are connected by a peelable heat seal proximate the perimeter of the thermoformed tray.

10. A packaged product according to claim 9 further comprising a barrier layer in the lid.

11. A packaged product according to claim 9 or 10 wherein
a) the polyvinyl chloride polymer is in direct contact with the heat sealable product contact layer, and
b) the polyvinyl chloride polymer releases from the heat sealable product contact layer, wherein "the polyvinyl chloride polymer releases from the heat sealable product contact layer" means that there is no tackiness between the product and the heat sealable product contact layer.

12. A packaged product according to claim 9, 10 or 11 wherein the barrier layer of the thermoformed tray comprises ethylene vinyl alcohol copolymer.

13. A packaged product according to claim 9, 10, 11 or 12 wherein the cyclic olefin copolymer of the heat sealable product contact layer of the thermoformed tray has a glass transition temperature of at least 40°C as determined by differential scanning calorimetry.

14. A packaged product comprising:
a) at least one packaging component comprising a heat sealable product contact layer comprising a cyclic olefin copolymer, the cyclic olefin copolymer having a glass transition temperature of at least 40°C as determined by differential scanning calorimetry, and
b) a product comprising flexible polyvinyl chloride polymer, wherein the flexible polyvinyl chloride comprises plasticizer, the polyvinyl chloride polymer being in direct contact with the heat sealable product contact layer, wherein the product releases from the heat sealable product contact layer after the packaged product is aged for at least 5 days at 55°C and 90% RH, wherein "the product releases from the heat sealable product contact layer" means that there is no tackiness between the product and the heat sealable product contact layer.

15. A packaged product according to claim 14 in which the packaged product is hermetically sealed.

## Patentansprüche

1. Verpacktes Produkt, umfassend:
a) eine thermogeformte Schale, die ein cyclisches Olefincopolymer umfasst, das eine Glasübergangstemperatur von mehr als 40 °C, wie durch dynamische Differenzkalorimetrie bestimmt, aufweist, und
b) ein Produkt, das flexibles Polyvinylchlorid umfasst, wobei das flexible Polyvinylchlorid Weichmacher umfasst, wobei zumindest der flexible Polyvinylchloridanteil des Produkts in direktem Kontakt mit dem cyclischen Olefincopolymer ist,
wobei, nachdem das verpackte Produkt zumindest 5 Tage bei 55 °C und 90 % RH gelagert wurde, das Produkt aus dem cyclischen Olefincopolymer freigegeben wird, wenn die Schale umgekehrt wird, was bedeutet, dass ohne jegliche physikalische Zwangsmittel das Gewicht des Produkts ausreicht, um das Produkt nach dem Umkehren der Verpackungskomponente von dem cyclischen Olefincopolymer der Produktkontaktfläche wegzubewegen.

2. Verpacktes Produkt nach Anspruch 1, das außerdem einen Deckel umfasst, der mit der thermogeformten Schale verbunden ist.

3. Verpacktes Produkt nach Anspruch 2, wobei der Deckel und die thermogeformte Schale durch ein Heißsiegel verbunden sind.

4. Verpacktes Produkt nach einem der vorhergehenden Ansprüche, wobei mindestens einer von dem Deckel oder der thermogeformten Schale ein atmungsaktives Material umfasst, das den Eintritt eines sterilisierenden Gases erlaubt, aber die Übertragung von Bakterien verhindert.

5. Verpacktes Produkt nach einem der vorhergehenden Ansprüche, wobei das verpackte Produkt hermetisch versiegelt ist.

6. Verpacktes Produkt nach Anspruch 1, wobei die thermogeformte Schale ferner ein Barrierepolymer umfasst, das dem verpackten Produkt Schutz bietet.

7. Verpacktes Produkt nach Anspruch 1, wobei das Produkt ein medizinisches Produkt ist.

8. Verpacktes Produkt nach Anspruch 7, wobei das Produkt sterilisiert wurde.

9. Verpacktes Produkt, das einen Deckel, eine thermogeformte Schale und ein Produkt umfasst, wobei
a) das Produkt zwischen dem Deckel und der thermogeformten Schale angeordnet ist und flexibles Polyvinylchloridpolymer umfasst, wobei das flexible Polyvinylchlorid Weichmacher umfasst;
b) die thermogeformte Schale umfasst:
i)eine heißsiegelbare Produktkontaktschicht, die ein cyclisches Olefincopolymer umfasst;
ii) zumindest eine Barriereschicht, die Materialien umfasst, die den verpackten Produkten Schutz bieten;
iii) zumindest eine innere Schicht, die Polyamid umfasst, wobei die zumindest eine innere Schicht an die Barriereschicht angrenzt; und
iv) eine Schicht, die Polyester umfasst, die größer als 75 % der Gesamtdicke der thermogeformten Schale ist; und
c) der Deckel und die thermogeformte Schale durch ein abziehbares Heißsiegel in der Nähe des Umfangs der thermogeformten Schale verbunden sind.

10. Verpacktes Produkt nach Anspruch 9, das ferner eine Barriereschicht in dem Deckel umfasst.

11. Verpacktes Produkt nach Anspruch 9 oder 10, wobei
a) das Polyvinylchloridpolymer in direktem Kontakt mit der heißsiegelbaren Produktkontaktschicht steht; und
b) das Polyvinylchloridpolymer von der heißsiegelbaren Produktkontaktschicht freigegeben wird, wobei "das Polyvinylchloridpolymer von der heißsiegelbaren Produktkontaktschicht freigegeben wird" bedeutet, dass keine Klebrigkeit zwischen dem Produkt und der heißsiegelbaren Produktkontaktschicht besteht.

12. Verpacktes Produkt nach Anspruch 9, 10 oder 11, wobei die Barriereschicht der thermogeformten Schale EthylenVinylalkohol-Copolymer umfasst.

13. Verpacktes Produkt nach Anspruch 9, 10, 11 oder 12, wobei das cyclische Olefincopolymer der heißsiegelbaren Produktkontaktschicht der thermogeformten Schale eine Glasübergangstemperatur von zumindest 40 °C, bestimmt durch dynamische Differenzkalorimetrie, aufweist.

14. Verpacktes Produkt, umfassend:
a) zumindest eine Verpackungskomponente, die eine heißsiegelbare Produktkontaktschicht umfasst, die ein cyclisches Olefincopolymer umfasst, wobei das cyclische Olefincopolymer eine Glasübergangstemperatur von zumindest 40 °C, wie durch dynamische Differenzkalorimetrie bestimmt, aufweist, und
b) ein Produkt, das das flexible Polyvinylchloridpolymer umfasst, wobei das flexible Polyvinylchlorid Weichmacher umfasst, wobei das Polyvinylchloridpolymer in direktem Kontakt mit der heißsiegelbaren Produktkontaktschicht steht, wobei das Produkt von der heißsiegelbaren Produktkontaktschicht freigegeben wird, nachdem das verpackte Produkt zumindest 5 Tage bei 55 °C und 90 % RH gealtert wurde, wobei "das Produkt von der heißsiegelbaren Produktkontaktschicht freigegeben wird" bedeutet, dass keine Klebrigkeit zwischen dem Produkt und der heißsiegelbaren Produktkontaktschicht besteht.

15. Verpacktes Produkt nach Anspruch 14, wobei das verpackte Produkt hermetisch versiegelt ist.

## Revendications

1. Produit emballé comprenant :
a) un plateau thermoformé comprenant un copolymère d'oléfine cyclique qui a une température de transition vitreuse supérieure à 40 °C, telle que déterminée par calorimétrie différentielle à balayage, et
b) un produit comprenant du polychlorure de vinyle flexible, dans lequel le polychlorure de vinyle flexible comprend un plastifiant, au moins la portion de polychlorure de vinyle flexible du produit étant en contact direct avec le copolymère d'oléfine cyclique,
dans lequel, après que le produit emballé a été stocké pendant au moins 5 jours à 55 °C et 90 % HR, le produit se détache du copolymère d'oléfine cyclique lorsque le plateau est retourné, ce qui signifie qu'en l'absence de tout moyen physique de contrainte, le poids du produit est suffisant pour éloigner le produit du copolymère d'oléfine cyclique de la surface de contact avec le produit après que le composant d'emballage a été retourné.

2. Produit emballé selon la revendication 1, comprenant également un couvercle relié au plateau thermoformé.

3. Produit emballé selon la revendication 2, dans lequel le couvercle et le plateau thermoformé sont reliés par un thermoscellage.

4. Produit emballé selon l'une quelconque des revendications précédentes, dans lequel au moins l'un parmi le couvercle ou le plateau thermoformé comprend un matériau respirant, qui permet l'entrée d'un gaz stérilisant mais empêche la transmission de bactéries.

5. Produit emballé selon l'une quelconque des revendications précédentes, dans lequel le produit emballé est scellé hermétiquement.

6. Produit emballé selon la revendication 1, dans lequel le plateau thermoformé comprend en outre un polymère barrière qui assure la protection du produit emballé.

7. Produit emballé selon la revendication 1, dans lequel le produit est un produit médical.

8. Produit emballé selon la revendication 7, dans lequel le produit a été stérilisé.

9. Produit emballé comprenant un couvercle, un plateau thermoformé et un produit, dans lequel
a) le produit est situé entre le couvercle et le plateau thermoformé et comprend un polymère de polychlorure de vinyle flexible, dans lequel le polychlorure de vinyle flexible comprend un plastifiant ;
b) le plateau thermoformé comprend :
i)une couche de contact de produit thermoscellable comprenant un copolymère d'oléfine cyclique ;
ii) au moins une couche barrière constituée de matériaux qui assurent la protection des produits emballés ;
iii) au moins une couche interne comprenant du polyamide, l'au moins une couche interne étant adjacente à la couche barrière ; et
iv) une couche comprenant un polyester qui est supérieur à 75 % de l'épaisseur totale du plateau thermoformé ; et
c) le couvercle et le plateau thermoformé sont reliés par un joint thermique pelable à proximité du périmètre du plateau thermoformé.

10. Produit emballé selon la revendication 9, comprenant en outre une couche barrière dans le couvercle.

11. Produit emballé selon la revendication 9 ou 10, dans lequel
a) le polymère de polychlorure de vinyle est en contact direct avec la couche de contact de produit thermoscellable ; et
b) le polymère de polychlorure de vinyle se détache de la couche de contact avec le produit thermoscellable, dans lequel « le polymère de polychlorure de vinyle se détache de la couche de contact avec le produit thermoscellable » signifie qu'il n'existe pas de pouvoir adhésif entre le produit et la couche de contact de produit thermoscellable.

12. Produit emballé selon la revendication 9, 10 ou 11, dans lequel la couche barrière du plateau thermoformé comprend un copolymère d'éthylène et d'alcool vinylique.

13. Produit emballé selon la revendication 9, 10, 11 ou 12, dans lequel le copolymère d'oléfine cyclique de la couche de contact de produit thermoscellable du plateau thermoformé a une température de transition vitreuse d'au moins 40 °C, telle que déterminée par calorimétrie différentielle à balayage.

14. Produit emballé comprenant :
a) au moins un composant d'emballage comprenant une couche de contact de produit thermoscellable comprenant un copolymère d'oléfine cyclique, le copolymère d'oléfine cyclique ayant une température de transition vitreuse d'au moins 40 °C, telle que déterminée par calorimétrie à balayage différentiel, et
b)un produit comprenant un polymère de polychlorure de vinyle flexible, dans lequel le polychlorure de vinyle flexible comprend un plastifiant, le polymère de polychlorure de vinyle étant en contact direct avec la couche de contact de produit thermoscellable, dans lequel le produit se détache de la couche de contact de produit thermoscellable après que le produit emballé a été vieilli pendant au moins 5 jours à 55 °C et 90 % HR, dans lequel « le produit se détache de la couche de contact de produit thermoscellable » signifie qu'il n'existe pas de pouvoir adhésif entre le produit et la couche de contact de produit thermoscellable.

15. Produit emballé selon la revendication 14, dans lequel le produit emballé est scellé hermétiquement.
